(19) **Europäisches Patentamt**
**European Patent Office**
**Office européen des brevets**

(11) Publication number: **0 525 167 B1**

(12) **EUROPEAN PATENT SPECIFICATION**

(45) Date of publication of patent specification: **13.09.95**   (51) Int. Cl.[6]: **A61K 9/127**

(21) Application number: **92906507.6**

(22) Date of filing: **30.10.91**

(86) International application number:
**PCT/US91/08111**

(87) International publication number:
**WO 92/14445 (03.09.92 92/23)**

(54) **SUSTAINED DRUG RELEASE THROUGH TOPICAL APPLICATION OF BIOADHESIVE LIPOSOMES.**

(30) Priority: **14.02.91 US 655879**

(43) Date of publication of application:
**03.02.93 Bulletin 93/05**

(45) Publication of the grant of the patent:
**13.09.95 Bulletin 95/37**

(84) Designated Contracting States:
**AT BE CH DE DK ES FR GB IT LI LU NL SE**

(56) References cited:
**WO-A-90/09782**

**BIOCHEMICAL AND BIOPHYSICAL RE-SEARCH COMMUNICATIONS vol. 160, no. 2, 28 April1989, DULUTH, MINN.(US) pages 732 - 736; Y. ISHII ET AL.: 'preparation of egf label-ed liposomes and their uptake by hepatocytes'**

**PATENT ABSTRACTS OF JAPAN vol. 9, no. 74 (C-273)(1797) 3 April 1985**

**G. GREGORIADIS 'liposome technology ,vol**

**iii' 1984 , CRC PRESS, INC. , BOCARATON (US)**

(73) Proprietor: **BAXTER INTERNATIONAL INC.**
**One Baxter Parkway**
**Deerfield, IL 60015 (US)**

(72) Inventor: **MARGALIT, Rimona**
**52 Zabotinsky Street**
**53 318 Givataim (IL)**

(74) Representative: **MacGregor, Gordon**
**ERIC POTTER CLARKSON**
**St. Mary's Court**
**St. Mary's Gate**
**Nottingham, NG1 1LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

## BACKGROUND OF THE INVENTION

The present invention relates to a novel drug delivery system, particularly to microscopic drug delivery systems (MDDS) utilizing drug-encapsulating bioadhesive liposomes for topical and local drug administration.

Currently, the topical and local administration of a drug can be in its free form, dissolved or dispersed in a suitable diluent, or in a vehicle such as a cream, gel or ointment. Examples of therapeutic or designated targets for topical or local drug administration include burns; wounds; bone injuries; ocular, skin, intranasal and buccal infections; ocular chronic situations such as glaucoma; and topically and locally accessed tumors. Several difficulties exist with the topical or local administration of a drug in its free form. For example, short retention of the drug at the designated site of administration reduces the efficacy of the treatment and requires frequent dosing. Exposure of the free form drug to the biological environment in the topical or local region can result in drug degradation, transformation into inactive entities and nondiscriminating and uncontrollable distribution of the drug. Such degradation and uncontrollable distribution of the drug can result in toxicity issues, undesirable side effects and loss of efficacy.

Microscopic drug delivery systems (MDDS) have been developed to overcome some of the difficulties associated by free drug administration. Drug-loaded MDDS can perform as sustained or controlled release drug depots. By providing a mutual protection of the drug and the biological environment, MDDS reduces drug degradation or inactivation. As a controlled drug release, MDDS improves drug efficacy and allows reduction in the frequency of dosing. Since the pharmacokinetics of free drug release from depots of MDDS are different than from directly-administered drug, MDDS provides an additional measure to reduce toxicity and undesirable side effects.

MDDS is divided into two basic classes: particulate systems, such as cells, microspheres, viral envelopes and liposomes; or nonparticulate systems which are macromolecules such as proteins or synthetic polymers. Liposomes have been studied as drug carriers and offer a range of advantages relative to these other MDDS systems. Composed of naturally-occurring materials which are biocompatible and biodegradable, liposomes are used to encapsulate biologically active materials for a variety of purposes. Having a variety of layers, sizes, surface charges and compositions, numerous procedures for liposomal preparation and for drug encapsulation within them have been developed, some of which have been scaled up to industrial levels.

Liposomes can be designed to act as sustained release drug depots and, in certain applications, aid drug access across cell membranes. Their ability to protect encapsulated drugs and various other characteristics make liposomes a popular choice in developing MDDS, with respect to the previous practices of free drug administration.

Despite the potential for improved drug delivery, utilization of drug-encapsulating does pose some difficulties. These difficulties include targeting, retention and stability in circulation, potential of toxicity upon chronic administration and inability to extravasate. Liposomes administered topically or locally do not possess the ability to be retained at the designated target site and therefore, may perform no better than topically or locally administered free drug. In recent years, attempt have been made to couple recognizing substances with liposomes to confer target specificity to the liposomes, namely antibodies, glycoproteins and lectins. Difficulties are presented when utilizing these recognizing substances. For example, antibodies can be patient specific and, therefore, add cost to the drug therapy.

It is known that specific receptors are present in designated targets. For example, epidermal growth factor (EGF) receptors are present in burns, wounds, ocular and dermal infections and tumors. Membrane-embedded mucin, present in certain mucinous carcinoma, are target sites for bioadhesive agents. Similarly, occular, intranasal and buccal targets have mucosal surfaces with which various bioadhesive agents have been shown to interact. However, these interactions do not offer any therapeutic value.

The microenvironment surrounding the surface of a bioadhesive liposome is a matrix of the three following components: (a) the bioadhesive ligands; (b) water molecules which behave different than in bulk water or at the surface of regular liposomes; and (c) ions, at distributions different than in the bulk solvent or at the surface of regular liposomes. This microenvironment, which is specific to the bioadhesive liposomes, is termed "the bioadhesive layer."

The modification of regular liposomes, that renders them bioadhesive, could interfere with the ability of the liposomes to act as sustained-release drug delivery systems. Such interference could take two directions: (1) drug release from the bioadhesive liposomes could be slower compared to similar regular liposomes; or, (2) drug release from bioadhesive liposomes could be faster compared to similar regular liposomes. A level of interference that amounts to minor changes in the rates of drug release from bioadhesive compared to regular liposomes is tolerable and acceptable. Levels of interference which correspond to major changes, such as orders of magnitude, in the rates of drug release from the bioadhesive compared to the regular liposomes are unacceptable. In the latter case, the bioadhesive liposomes might not be suitable for the designated applications.

Biochemical and Biophysical Research Communication, vol. 160, no.2, 1989, p.732-736 discloses liposomes containing a drug, wherein epidermal growth factor (EGF) is covalently linked to the surface of the liposomes and acts as a targeting moiety for targeting the liposomes to a cell. This article discloses that the liposomes are taken up by the target cells (probably by endocytosis), which makes the liposomes useful for delivery of a dose of an anti-cancer drug.

WO-A-9009782 discloses a liposome having EGF electrostatically adsorbed to its surface. The EGF is a drug which is readily released at the target site. WO-A-9009782 also discloses the use of a liposome composition in gel form for prolonging retention of the liposomes at the target site.

It has been learned that modifying regular liposomes by covalently anchoring certain recognizing substances to the liposomal surface creates a "bioadhesive" liposome with target specificity and retention. The recognizing substances are molecules which can be utilized as an adhesive or glue attaching a drug delivery system onto a target area.

G. Gregoriades, "Liposome Technology", 1984, p.76-90 discloses a liposome having anti-collagen or fibronectin covalently bonded to its surface to act as a targeting moiety for sites of endothelial injury. The liposome contains a drug for slow release.

To perform effectively, the topical or local administration of drug-encapsulating liposomes should have specificity for and the ability to adhere to the designated target area and should facilitate drug access to intracellular sites. Currently available liposomes and other MDDS systems do not meet these performance requirements of topical and local drug administration.

The present invention does meet these requirements and is set out in claim 1, whose pre-characterising part is based on the Gregoriades article and is directed to a microscopic delivery system for the sustained release of a substance comprising a liposome component, a substance encapsulated by the liposome component and a target recognising component covalently bonded to the liposomal surface the liposome component having a permeability that allows sustained substance release.

The distinguishing features of the present invention are set out in the characterising part of Claim 1, which is characterised in that the target recognising component is selected from collagen, gelatin and hyaluronic acid.

The present invention also provides use in the manufacture of a liposomal composition encapsulating a substance, for the purpose of adhering to a topical target site for sustained release of the substance, of a target-recognizing component covalently bonded to the liposomal surface and selected from gelatin, collagen, hyaluronic acid and epidermal growth factor.

Preferably, the "bioadhesive" recognizing substances can perform either through receptor mechanisms or through adhesions to components of the extracellular matrix. Regardless of the specific mechanism of adhesion, these recognizing substances are referred to as "bioadhesive recognizing substances" based on their common end result.

Through covalent anchoring, the bioadhesive recognizing substances become an integral part of the liposome, yet remain accessible to the interaction counterpart at the target site. They endow the liposome and encapsulated drug with the ability to adhere to the target site. The modification of the liposomes has been found not to interfere with the drug delivery from the intact liposomes. Interference of drug delivery can occur either through extensive slowing down of the efflux of drug from the liposome or through extensive acceleration of the efflux of drug, making the delivery similar to that of free drug administration. Hence, "bioadhesive" liposomes have been developed which are target adherent, sustained release drug depots.

## DETAILED DESCRIPTION

According to the present invention, bioadhesive, drug-encapsulating liposomes can bind and deliver an encapsulated drug to cell cultures having receptors or extracellular matrix which accommodate the recognizing substance bonded to the liposome. Liposomes, in particular, multilamellar vesicles (MLV), microemulsified liposomes (MEL) or large unilamellar vesicles (LUVET), each containing phosphatidylethanolamine (PE), have been prepared by established procedures. The bioadhesive recognizing substances of the present invention, each of which have been accepted for human use, include epidermal growth factor (EGF), hyaluronic acid (HA), gelatin and collagen. Each of these recognizing substances have a biological origin and are biodegradable and biocompatible. Further, these recognizing substances have functional residues which can be utilized in covalent anchoring to the regular liposomal surfaces.

Depending on solubility, drugs are introduced via the aqueous swelling solution or via the initial lipid/organic solvent solution for encapsulation in regular liposomes. Where desired, separation from unencapsulated drug can be done by centrifugation, dialysis or column chromatography. For drug encapsulation in bioadhesive liposomes, drug-encapsulating PE-containing liposomes are prepared and the liposomes are then modified by the bioadhesive recognizing substances as described. The liposomes are not separated from excess unencapsulated drug prior to binding of the recognizing substance, in order to minimize drug loss in the course of removing and washing excess recognizing substance and reagent.

Upon topical or local administration of bioadhesive, drug-encapsulating liposomes, the drug would diffuse from intact liposomes under the power of its electrochemical gradients, between the various pools in which it is located, and the external media. To determine the kinetic properties of drug release in such a situation, the bioadhesive, drug-encapsulating liposomes were studied by dialysis. A liposome/drug system is enclosed in a dialysis sac and is immersed in the desired medium, typically a buffer, under unidirectional, outward flux conditions. The accumulated drug in the dialysate is sampled at desired intervals and the liposome/drug system is assayed at zero time and at the time chosen for the termination of the experiment.

For the evaluation of the acceptability of the bioadhesive liposomes as sustained release drug delivery systems, data from both free liposomes and bioadhesive liposomes is analyzed according Mechanism 1, detailed below. Additional fine-tuning of the kinetics, aimed at resolving between surface-adsorbed and encapsulated drug is possible and can be performed by subjecting data from the bioadhesive liposomes to analysis according to Mechanism 2, detailed below.

At zero time, for data processing according to Mechanism 1, the drug within the dialysis sac is distributed between two pools. One pool is that of free, unencapsulated drug, the other pool is that of the liposome-associated drug. The diffusion of drug from each pool follows a single first-order process and both processes are independent of each other. Consequently, the overall rate of diffusion of drug from the sac into the dialysate can be described by two parallel first-order processes, one for the unencapsulated drug, the other for the liposome-associated drug. Denoting the fraction of the total drug in the system, which is present in the dialysate at time $= t$ as "f", the following equation (Mechanism 1) gives the quantitative relationship between time (the free variable) and f (the dependent variable):

$$f = f_1 * (1-\exp(-k_1 t)) + f_2 * (1-\exp(-k_2 t))$$

where $k_1$ and $k_2$ are the rate constants for the diffusion of the unencapsulated and liposome-associated drug, respectively, and $f_1$ and $f_2$ are the initial (i.e., at zero time) distributions (in fractions) of the total drug in the system, between the unencapsulated and liposome-associated pools, respectively, $f_1$ and $f_2$ sum to unity.

For the regular liposomes, the term "liposome-associated drug" corresponds to drug which is encapsulated within the liposome. Consequently, $k_2$ is the rate constant of the encapsulated drug and $f_2$ is the fraction of the total drug in the system, which is encapsulated in the liposome preparation (prior to initiation of the kinetic experiment).

For the bioadhesive liposomes, the term "liposome-associated drug" is the sum of the drug encapsulated within the liposome and of the drug adsorbed into the surface of the liposome within the bioadhesive layer. Consequently, $k_2$ is a combination of the rate constants for drug diffusion from the encapsulated and adsorbed locations $f_2$ is the sum of the fractions of the total drug in the system which are encapsulated and adsorbed in the liposome preparation (prior to initiation of the kinetic experiment).

At zero time, for data processing according to Mechanism 2, the drug within the dialysis sac is distributed between three pools. One pool is that of unencapsulated drug. The second and third pools are of liposome-associated drug, as defined in Mechanism 1 for the bioadhesive liposome, but are now separated into the pool of drug which is surface-adsorbed within the bioadhesive layer and the pool of drug which is encapsulated within the liposome. The diffusion of drug from each pool follows a single first-order process and all three processes are independent of each other. Consequently, the overall rate of diffusion of drug from the sac into the dialysate can be described by three parallel first-order processes. One for the unencapsulated drug, one for the surface-adsorbed drug and one for the encapsulated drug. Denoting the fraction of the total drug in the system, which is present in the dialysate at time $= t$ as "f", the following equation (Mechanism 2) gives the quantitative relationship between time (the free variable) and f (the dependent variable):

4

$$f = f_1{}^*(1\text{-}exp(\text{-}k_1 t)) \ + \ f_2{}^*(1\text{-}exp(\text{-}k'_2 t)) + f_2{}^*(1\text{-}exp(\text{-}k_2 t))$$

where $k_1$, $k'_2$ and $k_2$ are the rate constants for the diffusion of the unencapsulated, adsorbed encapsulated drug, respectively, $f_1$, $f'_2$ and $f_2$ are the initial distributions (in fractions) of the total drug in the system, among unencapsulated, adsorbed and encapsulated drug, respectively, and sum to unity. $f_2$ is the fraction of the total drug in the system, which is encapsulated in the liposome preparation (prior to initiation of the kinetic experiment).

Example 1

Multilamellar, progesterone-encapsulating liposomes were prepared. The progesterone was encapsulated in the liposomes in the course of liposome preparation, by its addition to the initial lipid/organic solvent solution. The liposomes were modified by the binding of gelatin as previously described. The progesterone-containing gelatin-modified liposomes, at a lipid concentration of 15 mM were put into a dialysis sac and the kinetics of progesterone efflux were studied, as described above. A similar experiment, under the same conditions was performed with progesterone-containing regular liposomes from the same original liposome batch. The data of both experiments were processed according to Mechanism 1, described above. For the regular liposomes, it was found that $f_2$, the fraction of the total progesterone in the system which is encapsulated within the liposomes is 70% and that $k_2$, the rate constant of the diffusion of the encapsulated progesterone, is $3.1 \times 10^{-3}$ hours$^{-1}$. For the bioadhesive liposomes, it was found that $f_2$, the fraction of the total progesterone in the system which is liposome-associated (that is, both encapsulated and adsorbed) is 83% and that the $k_2$, the corresponding rate constant, is $7.1 \times 10^{-3}$ hours$^{-1}$. The finding that the increase in $k_2$ for the gelatin-modified liposomes compared to that of the regular liposomes is by a factor of two alone, shows that modifying the liposomes by bioadhesive recognizing substances does not significantly interfere with the rate constant of drug efflux from liposomes.

Example 2

Extruded, large unilamellar leucine-enkephalin-encapsulating liposomes were prepared. The leucine-enkephalin was encapsulated in the liposomes in the course of liposome preparation, by its addition to the aqueous swelling solution. The liposomes were modified by the binding of HA as previously described. The leucine-enkephalin-containing HA-modified liposomes, at a lipid concentration of 15 mM, were put into a dialysis sac and the kinetics of leucine-enkephalin efflux were studied, as described above. A similar experiment, under the same conditions was performed with leucine-enkephalin-containing regular liposomes, from the same original liposome batch. The data of both experiments were processed according to Mechanism 1, described above. For the regular liposomes, it was found that $f_2$, the fraction of the total leucine-enkephalin in the system which is encapsulated within the liposomes is 47% and that $k_2$, the rate constant of the diffusion of the leucine-enkephalin, is 0.014 hours$^{-1}$. For the bioadhesive liposomes, it was found that $f_2$, the fraction of the total leucine-enkephalin in the system which is liposome-associated is 67% and that the corresponding magnitude of $k_2$, is 0.038 hours$^{-1}$. The finding that the increase in $k_2$ for the HA-modified liposomes, compared to that of the regular liposomes is by a factor of three alone, already shows that modifying the liposomes by bioadhesive recognizing substances does not significantly interfere with the rate constant of drug efflux from liposomes. This conclusion is further strengthened by analysis of the efflux of leucine-enkephalin from the bioadhesive liposomes according to Mechanism 2. Such analysis showed $f_2$, the fraction of the total leucine-enkephalin in the system which is encapsulated within the bioadhesive liposome to be 36%. Also, $k_2$, the rate constant of diffusion of encapsulated leucine-enkephalin from these bioadhesive liposomes was found to be 0.014 hours$^{-1}$. This is the same magnitude determined and listed above for the rate constant of diffusion of encapsulated leucine-enkephalin from regular liposomes.

From this effort, it is seen that modifying the liposomes by bioadhesive recognizing substances does not significantly interfere with the rate constant of drug efflux from the liposomes. Gelatin and HA recognizing substances used in the disclosed examples could be substituted with growth factor or collagen from either natural or synthetic sources.

**Claims**

1. A microscopic delivery system for the sustained release of a substance comprising a liposome component, a substance encapsulated by the liposome component and a target-recognizing component covalently bonded to the liposomal surface the liposome component having a permeability that allows sustained substance release, characterised in that the target-recognizing component is selected from gelatin, collagen and hyaluronic acid.

2. The delivery system of claim 1 wherein the liposome component is selected from the group consisting of multilamellar vesicles, microemulsified liposomes and large unilamellar vesicles.

3. The delivery system of claim 1 or 2 wherein the liposome component includes phosphatidylethanolamine.

4. The delivery system of any preceding claim wherein the target recognizing component confers to the liposome component target specificity for and retention for a designated cellular target site for the release of the substance with the uninterfered efflux of the substance from the liposome component.

5. The delivery system of claim 1, 2 or 3 wherein the target recognizing component confers target specificity to and retention for a designated cellular target site for the release of the substance by a mechanism of adhesion through receptor mechanisms at the cellular target site.

6. The delivery system of Claim 1, 2 or 3 wherein the target recognizing component confers target specificity to and retention for a designated cellular target site for the release of the substance by a mechanism of adhesion through associations with components within an extracellular matrix at the cellular target site.

7. The delivery system of any preceding Claim where the liposome component and the target recognizing component are covalently linked by a crosslinking reagent.

8. Use in the manufacture of a liposomal composition encapsulating a substance, for the purpose of adhering to a topical target site for sustained release of the substance, of a target-recognizing component covalently bonded to the liposomal surface and selected from gelatin, collagen, hyaluronic acid and epidermal growth factor.

9. Use according to Claim 8, wherein the delivery system is according to any one of Claims 2 to 7.


**Patentansprüche**

1. Mikroskopisches Abgabesystem für die andauernde Freigabe einer Substanz, die folgendes aufweist:
   eine Liposomkomponente,
   eine Substanz, die von der Liposomkomponente eingekapselt ist, und
   eine Zielerkennungskomponente, die an die Liposomoberfläche kovalent gebunden ist, wobei die Liposomkomponente eine Permeabilität hat, die eine andauernde Freigabe der Substanz ermöglicht,
   dadurch gekennzeichnet,
   daß die Zielerkennungskomponente aus Gelatine, Collagen und Hyaluronsäure ausgewählt ist.

2. Abgabesystem nach Anspruch 1,
   wobei die Liposomkomponente aus der Gruppe ausgewählt ist, die aus multilamellaren Vesikeln, mikroemulgierten Liposomen und großen unilamellaren Vesikeln besteht.

3. Abgabesystem nach Anspruch 1 oder 2,
   wobei die Liposomkomponente Phosphatidylethanolamin aufweist.

4. Abgabesystem nach einem der vorhergehenden Ansprüche,
   wobei die Zielerkennungskomponente der Liposomkomponente eine Zielspezifität und eine Retention für eine bestimmte Zellzielstelle verleiht, um die Substanz mit ungestörtem Ausfluß der Substanz aus der Liposomkomponente freizugeben.

5. Abgabesystem nach Anspruch 1, 2 oder 3,
   wobei die Zielerkennungskomponente eine Zielspezifität und eine Retention für eine bestimmte Zellzielstelle verleiht, um die Substanz durch einen Haftmechanismus über Rezeptormechanismen an der Zellzielstelle freizugeben.

6. Abgabesystem nach Anspruch 1, 2 oder 3,
wobei die Zielerkennungskomponente eine Zielspezifität und eine Retention für eine bestimmte Zellzielstelle verleiht, um die Substanz durch einen Haftmechanismus über Assoziationen mit Komponenten in einer extrazellulären Matrix an der Zellzielstelle freizugeben.

7. Abgabesystem nach einem der vorhergehenden Ansprüche,
wobei die Liposomkomponente und die Zielerkennungskomponente durch ein quervernetzendes Reagens kovalent gebunden sind.

8. Verwendung einer Zielerkennungskomponente bei der Herstellung einer Liposomenzusammensetzung, die eine Substanz einkapselt, zum Zweck des Haftens an einer topischen Zielstelle für die andauernde Freigabe der Substanz, wobei die Zielerkennungskomponente an die Liposomoberfläche kovalent gebunden ist und aus Gelatine, Collagen, Hyaluronsäure und epidermalem Wachstumsfaktor ausgewählt ist.

9. Verwendung nach Anspruch 8,
wobei das Abgabesystem gemäß einem der Ansprüche 2 bis 7 ausgebildet ist.

**Revendications**

1. Système d'administration microscopique pour la libération retard d'une substance comprenant un constituant liposome, une substance encapsulée par le constituant liposome et un constituant de reconnaissance de cible, lié par liaison covalente à la surface des liposomes, le constituant liposome ayant une perméabilité qui permet une libération retard de la substance, caractérisé en ce que le constituant de reconnaissance de cible est choisi parmi la gélatine, le collagène et l'acide hyaluronique.

2. Système d'administration selon la revendication 1, dans lequel le constituant liposome est choisi parmi l'ensemble comprenant les vésicules multilamellaires, les liposomes micro-émulsifiés et les grosses vésicules unilamellaires.

3. Système d'administration selon la revendication 1 ou 2, dans lequel le constituant liposome comprend la phosphatidyléthanolamine.

4. Système d'administration selon l'une quelconque des revendications précédentes, dans lequel le constituant de reconnaissance de cible confère au constituant liposome une spécificité de cible vis-à-vis d'un site cible cellulaire désigné, et une rétention de ce site, pour permettre la libération de la substance, la substance sortant sans entrave du constituant liposome.

5. Système d'administration selon la revendication 1, 2 ou 3, dans lequel le constituant de reconnaissance de cible confère une spécificité de cible vis-à-vis d'un site cible cellulaire désigné, et une rétention de ce dernier, pour la libération de la substance par un mécanisme d'adhérence par l'intermédiaire de mécanismes récepteurs au niveau du site cible cellulaire.

6. Système d'administration selon la revendication 1, 2 ou 3, dans lequel le constituant de reconnaissance de cible confère une spécificité de cible vis-à-vis d'un site cible cellulaire désigné, et une rétention de ce dernier, pour la libération de la substance par un mécanisme d'adhérence par l'intermédiaire d'associations avec des constituants se trouvant dans une matrice extracellulaire au niveau du site cible cellulaire.

7. Système d'administration selon l'une quelconque des revendications précédentes, dans lequel le constituant liposome et le constituant de reconnaissance de cible sont liés par liaison covalente à l'aide d'un réactif de réticulation.

8. Utilisation, lors de la fabrication d'une composition liposomique encapsulant une substance, dans le but d'adhérer à un site cible topique, pour permettre une libération retard de la substance, d'un constituant de reconnaissance de cible lié par liaison covalente à la surface liposomique, et choisi parmi l'ensemble comprenant la gélatine, le collagène, l'acide hyaluronique et le facteur de croissance de l'épiderme.

9. Utilisation selon la revendication 8, dans laquelle le système d'administration est conforme à l'une quelconque des revendications 2 à 7.